# EUROPEAN PATENT APPLICATION

(11) **EP 2 883 539 A1**
(43) Date of publication of application: **17.06.2015**
(21) Application number: 14197346.1
(22) Date of filing: 11.12.2014
(51) Int. Cl.: A61K 9/20, A61K 9/24, A61K 31/4178, A61K 31/4422

(54) **Pharmaceutical combinations of olmesartan and amlodipine**

(30) Priority: 12.12.2013 TR 201314592
(71) Applicant: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Sezgin, Asiye, 34460 Istanbul (TR); Tombayoglu, Vildan, 34460 Istanbul (TR)
(74) Representative: Sevinç, Erkan

(57) **Abstract**

The present invention relates to pharmaceutical combination comprising olmesartan medoxomil, amlodipine or a pharmaceutically acceptable salt thereof and erythritol together with one or more pharmaceutically acceptable excipients. Particularly, the formulation comprises crospovidone as a disintegrant.

## Description

### Field of Invention

The present invention relates to pharmaceutical combination comprising olmesartan medoxomil, amlodipine or a pharmaceutically acceptable salt thereof and erythritol together with one or more pharmaceutically acceptable excipients. Particularly, the formulation comprises crospovidone as a disintegrant.

### Background of Invention

The active ingredient olmesartan medoxomil is a prodrug which is hydrolized to olmesartan during absorbtion in the gastrointestinal tract. Olmesartan is selective AT₁ subtype angiotensin II receptor antagonist.

Olmesartan medoxomil has a chemical name as 2,3-dihydroxy-2-butenyl 4-(1-hydroxy-1-methylethyl)-2-propyl-1-[p-(o-1 H-tetrazol-5-ylphenyl)benzyl]imidazole-5-carboxylate, cyclic 2,3-carbonate and its chemical structure is shown in the Formula I.

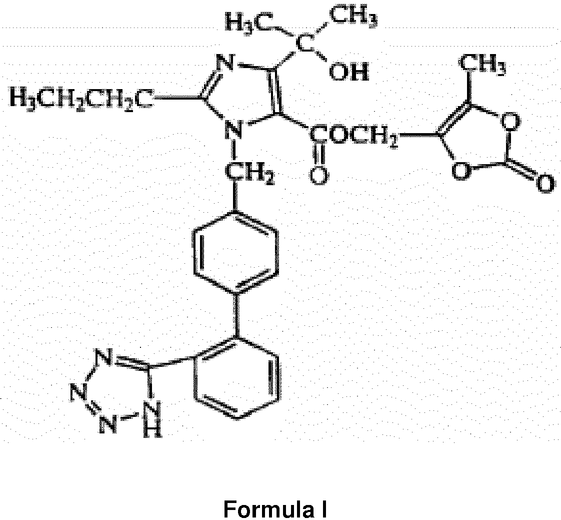

Olmesartan medoxomil is known from the US Patent No. 5,616,599 and is available in the market as film-coated tablet formulations. It is administered orally in a therapeutic dose of 5 mg, 20 mg or 40 mg and is indicated for the treatment of hypertension. It may be used alone or in combination with other antihypertensive agents.

Amlodipine is an orally-administered calcium channel blocker. Its chemical designation is (RS)-3-ethyl-5-methyl-2-(2-aminoethoxymethyl)-4-(2-chlorophenyl)-6-methyl-1,4-dihydropyridine-3,5-dicarboxylate with the following chemical structure of Formula II.

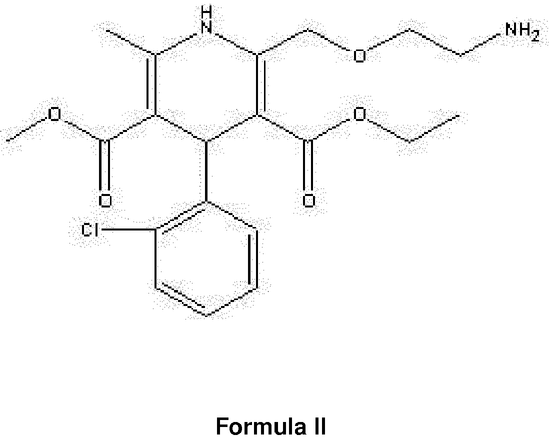

Amlodipine is presently available in the form of orally-administrable 5 to 10 mg tablets.

In addition, there have been various patent and patent applications in terms of combinations of olmesartan medoxomil and amlodipine. The patent application WO2007/001065 discloses a coated solid formulation comprising olmesartan, amlodipine and hydroxypropylcellulose, microcrystalline cellulose, lactose and magnesium stearate as excipients. According to this application, to perform wet granulation, lactose and HPMC is used. However, in the literature, it is disclosed that the use of lactose induces instability problems in amlodipine molecule with primary amino group. In application WO 2008/032107, to overcome this problem, a solid dosage formulation comprising olmesartan medoxomil, amlodipine and prejelatinized starch, silicified microcrystalline cellulose, croscarmellose sodium, magnesium stearate as excipients has been developed. This formulation is also in the market under the name of AZOR® and tablets are available in strengths of 20/5, 40/5, 20/10 and 40/10mg of olmesartan medoxomil to amlodipine.

Olmesartan medoxomil has solubility problem and amlodipine has stability problem. Therefore, it is difficult to combine these active agents in a fixed dose formulation that has desired dissolution profile. Well-directed processes are still required to formulate this pharmaceutical combination with high stability. In this invention, to increase the solubility and stability of combination of olmesartan medoxomil and amlodipine, erythritol was used as diluent. Furthermore, to further improve dissolution, crospovidone has been used as a disintegrant.

### Description of Invention

The present invention relates to pharmaceutical combination comprising olmesartan medoxomil, amlodipine or a pharmaceutically acceptable salt thereof and erythritol together with one or more pharmaceutically acceptable excipients. Particularly, the formulation comprises crospovidone as a disintegrant.

In another embodiment of the present invention is to obtain stable combination formulations with desired dissolution profiles for use in the treatment of hypertension.

According to another embodiment, pharmaceutical combination comprising olmesartan medoxomil, amlodipine or a pharmaceutically acceptable salt thereof and erythritol as diluent is in the oral solid dosage form.

In one embodiment, the amount of olmesartan medoxomil is between 0.1 - 15.0%, preferably 3.0 - 10.0% and more preferably it is 8.0 - 10.0% by weight of total formulation.

According to one embodiment, amlodipine or a pharmaceutically acceptable salt thereof presents in the pharmaceutical combination as amlodipine besylate.

In another embodiment, the amount of amlodipine besylate is between 0.1 - 15.0%, preferably 0.1 - 7.0% by weight of total formulation.

According to another embodiment, the ratio of olmesartan medoxomil to amlodipine is in the range of 0.1 to 10 (w/w) and preferably 0.1 to 6 (w/w).

In a fixed dose combination formulation, each active ingredient should have desired dissolution simultaneously. Solubility of active agents plays a prime role in controlling its dissolution from dosage form. Non-soluble active ingredients may cause undesired dissolution profile. Especially, in this combination, it is difficult to achieve desired dissolution profile for both olmesartan and amlodipin due to their differences on solubility. Olmesartan medoxomil is hydrophobic molecule that is insoluble in water. In some cases, while amlodipine has desired dissolution, olmesartan may be does not have. Therefore, it is very crucial to choose the suitable diluent that improves the solubility of olmesartan medoxomil.

On the other side, amlodipine besylate has stability problems with some excipients such as lactose used in solid pharmaceutical dosage forms. To perform wet granulation, in patent application WO2007/001065, lactose has been used in the formulation. However, amlodipine which has hygroscopic properties is unstable in a formulation comprising lactose.

Hygroscopicity is an important factor for stability of a pharmaceutical formulation comprising active agents that are affected by moisture such as amlodipine. Erythritol is a non-hygroscopic diluent. It has been found that, by using erythritol, while the chemical stability of amlodipine has been provided, solubility of olmesartan medoxomil also has been improved. For that reason, to obtain stability and desired dissolution properties, wet granulation has been performed by using erythritol.

In one embodiment, to increase solubility of combination, in the formulation of this present invention, erythritol presents in an amount of between 35.0 - 90.0%, preferably 35.0 - 70.0% and more preferably it is 40.0 - 55.0% by weight of total formulation.

In one embodiment, in this present pharmaceutical combination, one or more pharmaceutically acceptable excipient is crospovidone (Kollidon CL).

While using erythritol to enhance solubility of olmesartan, solubility of amlodipine may be decreased. In this invention, to further improve the dissolution of amlodipine, crospovidone has been used as a disintegrant. It is a non-hygroscopic "super disintegrant". Therefore, while it improves the dissolution, it also further improves the stability of these present combination formulations.

According to another embodiment, the amount of crospovidone is between 2.0 - 25.0%, preferably 2.0 - 15.0% and more preferably it is 9.0 - 12.0% by weight of total formulation.

In one embodiment, it has been surprisingly found that with the synergistic effect of erythritol and crospovidone, stability and dissolution problems have been overcome for the olmesartan medoxomil and amlodipine combination formulation.

According to these embodiments, dissolution test has been performed for the present combination formulation under the conditions as follows; in 900mL of pH 6.8 phosphate buffers at 50 rpm named as USP II (Paddle) and at 37°C ±0.5°C.

In further embodiment, the pharmaceutical combination comprises a diuretic. In this invention, hydrochlorothiazide has been used as a diuretic.

It is known that co-administrations of anti-hypertensives and diuretics are an effective therapy for the prevention or treatment of hypertension. Especially, thiazide diuretics are used in the treatment of hypertension, either alone or in combination with other antihypertensives. In this invention, pharmaceutical combination further comprises hydrochlorothiazide as a thiazide diuretic to enhance the effect of antihypertensives.

According to this embodiment, the amount of hydrochlorothiazide (HCTZ) is between 0.1 - 15.0%, preferably 1.0 - 10.0% and more preferably it is 2.5 - 6.5% by weight of total formulation.

According to another embodiment, the ratio of olmesartan medoxomil to hydrochlorothiazide is in the range of 0.1 to 10 (w/w) and preferably 0.1 to 4 (w/w).

According to another embodiment, the ratio of amlodipine besylate to hydrochlorothiazide is in the range of 0.1 to 10 (w/w) and preferably 0.1 to 4 (w/w).

Suitable disintegrants may include but not limited to sodium starch glycolate, microcrystalline cellulose, croscarmellose sodium, sodium carboxymethyl starch, soy polysaccharide, cross-linked alginic acid, crospovidone, copovidone, gellan gum, xanthan gum, calcium silicate or ion exchange resins or mixtures thereof; preferably they are sodium starch glycolate and microcrystalline cellulose.

Suitable binders may include but not limited to polyvinylpyrrolidone, polyethylene glycol, polyvinyl alcohol, polyvinyl acetate and their copolymers, cellulose derivatives such as hydroxypropyl methyl cellulose, carboxy methyl cellulose, methyl cellulose, microcrystalline cellulose, gelatin, polyvinylalcohol, carrageenan, guar gum and mixtures thereof; preferably it is polyvinylpyrrolidone.

Suitable glidants may include but not limited to colloidal silicon dioxide, talc, aluminium silicate and the like and mixtures thereof, preferably it is colloidal silicon dioxide. Suitable lubricants may include but not limited to sodium stearyl fumarate, magnesium stearate, polyethylene glycol (PEG), sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof, preferably it is sodium stearyl fumarate.

For the moisture protection, coating material may include but not limited polyvinylalcohol based films (Opadry 200), polyvinyl alcohol or copolymers or mixtures thereof (Opadry AMB), polyvinyl alcohol-polyethylene glycol copolymers (Kollicoat IR), hydroxypropyl methyl cellulose (HPMC), Ethylcellulose Dispersions (Surelease), Kerry-HPC, polyethylene glycol, polyvinylprolidone, polyvinylprolidone-vinyl acetate copolymer(PVP-VA), and all kinds of OpadryTM, as well as pigments, dyes, titanium dioxide and iron oxide, talk and polymethylmetacrylate copolymers (Eudragit), preferably it is Opadry 200.

In one embodiment, the pharmaceutical combination of this present invention is in the form of a monolayer, bilayer, trilayer, or a multilayer tablet.

According to this embodiment, the pharmaceutical combination of this present invention is in the form of a monolayer tablet.

According to the present invention, said pharmaceutical combinations are comprised of the following ingredients:
a. 0.1 - 15.0 % by weight of olmesartan medoxomil,
b. 0.1 - 15.0 % by weight of amlodipine besylate,
c. 35.0 - 90.0 % by weight of erythritol,
d. 2.0 - 25.0 % by weight of crospovidone (Kollidon CL),
e. 4.0 - 50.0 % by weight of microcrystalline cellulose,
f. 0.1 - 10.0 % by weight of polyvinylpyrrolidone (PVP 30)
g. 5.0 - 15.0 % by weight of sodium starch glycolate ,
h. 0.1 - 5.0 % by weight of colloidal silicon dioxide
i. 0.1 - 5.0 % by weight of sodium stearyl fumarate
j. 1.0 - 7.0 % by weight of coating.
   and,

a. 0.1 - 15.0 % by weight of olmesartan medoxomil,
b. 0.1 - 15.0 % by weight of amlodipine besylate,
c. 0.1 - 15.0% by weight of hydrochlorothiazide (HCTZ)
d. 35.0 - 90.0 % by weight of erythritol,
e. 2.0 - 25.0 % by weight of crospovidone (Kollidon CL),
f. 4.0 - 50.0 % by weight of microcrystalline cellulose,
g. 0.1 - 10.0 % by weight of polyvinylpyrrolidone (PVP 30)
h. 5.0 - 15.0 % by weight of sodium starch glycolate ,
i. 0.1 - 5.0 % by weight of colloidal silicon dioxide
j. 0.1 - 5.0 % by weight of sodium stearyl fumarate
k. 1.0 - 7.0 % by weight of coating.

In another embodiment, the pharmaceutical combination of this present invention is in the form of a bilayer tablet.

According to the present invention, said pharmaceutical combinations are comprised of the following ingredients in the form of a bilayer tablet:
In first layer;
   a. 0.1 - 15.0 % by weight of olmesartan medoxomil,
   b. 2.0 - 15.0 % by weight of crospovidone (Kollidon CL),
   c. 0.1 - 10.0 % by weight of polyvinylpyrrolidone (PVP 30)
   d. 35.0 - 90.0 % by weight of erythritol,
   e. 4.0 - 50.0 % by weight of microcrystalline cellulose,
   f. 0.1 - 5.0 % by weight of sodium stearyl fumarate
In second layer;
   g. 0.1 - 15.0 % by weight of amlodipine besylate,
   h. 2.0 - 15.0 % by weight of crospovidone (Kollidon CL),
   i. 4.0 - 50.0 % by weight of microcrystalline cellulose,
   j. 5.0 - 15.0 % by weight of sodium starch glycolate ,
   k. 0.1 - 5.0 % by weight of colloidal silicon dioxide
   l. 0.1 - 5.0 % by weight of sodium stearyl fumarate and,
   m. 1.0 - 7.0 % by weight of coating for the total tablet.
      and,
In first layer;
   a. 0.1 - 15.0 % by weight of olmesartan medoxomil,
   b. 2.0 - 15.0 % by weight of crospovidone (Kollidon CL),
   c. 0.1 - 10.0 % by weight of polyvinylpyrrolidone (PVP 30)
   d. 35.0 - 90.0 % by weight of erythritol,
   e. 4.0 - 50.0 % by weight of microcrystalline cellulose,
   f. 0.1 - 5.0 % by weight of sodium stearyl fumarate In second layer;
   g. 0.1 - 15.0 % by weight of amlodipine besylate
   h. 0.1 - 15.0% by weight of hydrochlorothiazide (HCTZ),
   i. 2.0 - 15.0 % by weight of crospovidone (Kollidon CL),
   j. 4.0 - 50.0 % by weight of microcrystalline cellulose,
   k. 5.0 - 15.0 % by weight of sodium starch glycolate ,
   l. 0.1 - 5.0 % by weight of colloidal silicon dioxide
   m. 0.1 - 5.0 % by weight of sodium stearyl fumarate and,
   n. 1.0 - 7.0 % by weight of coating for the total tablet.

### Example 1: Monolayer tablet

| **ingredient** | **amount %** |
|---|---|
| olmesartan medoxomil | 8 - 10% |
| amodipine besylate | 2 - 7% |
| erythritol | 40 - 55% |
| crospovidone | 9 - 12% |
| microcrystalline cellulose | 9 - 12% |
| polyvinylpyrrolidone | 1 -3% |
| sodium starch glycolate | 6 - 9% |
| colloidal silicon dioxide | 0.5 - 2 % |
| sodium stearyl fumarate | 1 - 3 % |
| coating | 2 - 4% |
| ethanol | q.s. |

| | |
|---|---|
| *q.s.: quantum sufficient* | |

The process of the formulations is carried out as follows: polyvinylprollidone solution is prepared in ethanol. A part of Crospovidone (Kollidon CL) is added to polyvinylprollidone solution. Olmesartan medoxomil and this solution are mixed in collete and further mixed. Olmesartan medoxomil mixture is added to a mixture that is comprised of a part of crospovidone, erythritol and microcrystalline cellulose and mixed, dried and sieved. On the other side, amlodipine besylate, a part of crospovidone, sodium starch glycolate and colloidal silicon dioxide are mixed and sieved. This amlodipine besylate mixture and olmesartan medoxomil mixture are mixed. Sodium stearyl fumarate is added total mixture and mixed while sieving. Final powder mixture are pressed into tablets and coated.

### Example 2: Monolayer tablet

| **ingredient** | **amount %** |
|---|---|
| olmesartan medoxomil | 8 - 10% |
| amodipine besylate | 2 - 7% |
| hydrochlorothiazide | 2.5 - 6.5 % |
| erythritol | 40 - 55% |
| crospovidon | 9 - 12% |
| microcrystalline cellulose | 9 - 12% |
| polyvinylpyrrolidone | 1 - 3% |
| sodium starch glycolate | 6 - 9% |
| colloidal silicon dioxide | 0.5 - 2 % |
| sodium stearyl fumarate | 1 - 3 % |
| coating | 2 - 4% |
| ethanol | q.s. |

| | |
|---|---|
| *q.s.: quantum sufficient* | |

The process of the formulations is carried out as follows: polyvinylprollidone solution is prepared in ethanol. A part of Crospovidone (Kollidon CL) is added to polyvinylprollidone solution. Olmesartan medoxomil and this solution are mixed in collete and further mixed. Olmesartan medoxomil mixture is added to a mixture that is comprised of a part of crospovidone, erythritol and microcrystalline cellulose and mixed, dried and sieved. On the other side, amlodipine besylate and hydrochlorothiazide (HCTZ), a part of crospovidone, sodium starch glycolate and colloidal silicon dioxide are mixed and sieved. This amlodipine besylate mixture and olmesartan medoxomil mixture are mixed. Sodium stearyl fumarate is added total mixture and mixed while sieving. Final powder mixture are pressed into tablets and coated.

### Example 3: Bilayer tablet

| **First layer** | **amount %** |
|---|---|
| olmesartan medoxomil | 8 - 10% |
| crospovidone | 2 - 15% |
| polyvinylpyrrolidone | 1 - 3% |
| erythritol | 40 - 55% |
| microcrystalline cellulose | 4 - 10% |
| sodium stearyl fumarate | 1 - 3 % |

| **Second layer** | **amount %** |
|---|---|
| amodipine besylate | 2 - 7% |
| crospovidone | 2 - 15% |
| microcrystalline cellulose | 4 - 10% |
| sodium starch glycolate | 6 - 9% |
| colloidal silicon dioxide | 0.5 - 2 % |
| sodium stearyl fumarate | 1 - 3 % |
| | |
| coating | 2 - 4% |
| ethanol | q.s. |

| | |
|---|---|
| *q.s.: quantum sufficient* | |

The process of the formulations is carried out as follows:

For the first layer: polyvinylprollidone solution is prepared in ethanol. A part of Crospovidone (Kollidon CL) is added to polyvinylprollidone solution and mixed. Olmesartan medoxomil and this solution are mixed in collete and further mixed. Olmesartan medoxomil mixture is added to a mixture that is comprised of a part of crospovidone, erythritol and microcrystalline cellulose and mixed, dried and sieved. Then, it is mixed with sodium stearyl fumarate.

For the second layer: amlodipine besylate, a part of crospovidone, sodium starch glycolate, and microcrystalline cellulose and colloidal silicon dioxide are mixed and sieved. Sodium stearyl fumarate is added to mixture and further mixed.
First layer and second layer are pressed into bilayer tablets and coated.

### Example 4: Bilayer tablet

| **First layer** | **amount %** |
|---|---|
| olmesartan medoxomil | 8 - 10% |
| crospovidone | 2 - 15% |
| polyvinylpyrrolidone | 1 - 3% |
| erythritol | 40 - 55% |
| microcrystalline cellulose | 4 - 10% |
| sodium stearyl fumarate | 1 - 3 % |

| **Second layer** | **amount %** |
|---|---|
| amodipine besylate | 2 - 7% |
| hydrochlorothiazide | 2.5 - 6.5 % |
| sodium starch glycolate | 6 - 9% |
| crospovidone | 2 - 15% |
| microcrystalline cellulose | 4 - 10% |
| colloidal silicon dioxide | 0.5 - 2 % |
| sodium stearyl fumarate | 1 - 3 % |
| | |
| coating | 2 - 4% |
| ethanol | q.s. |

| | |
|---|---|
| *q.s.: quantum sufficient* | |

The process of the formulations is carried out as follows:
For the first layer: polyvinylprollidone solution is prepared in ethanol. A part of Crospovidone (Kollidon CL) is added to polyvinylprollidone solution and mixed. Olmesartan medoxomil and this solution are mixed in collete and further mixed. Olmesartan medoxomil mixture is added to a mixture that is comprised of a part of crospovidone, erythritol and microcrystalline cellulose and mixed, dried and sieved. Then, it is mixed with sodium stearyl fumarate.

For the second layer: amlodipine besylate, hydrochlorothiazide (HCTZ), a part of crospovidone, sodium starch glycolate, and microcrystalline cellulose and colloidal silicon dioxide are mixed and sieved. Sodium stearyl fumarate is added to mixture and further mixed.
First layer and second layer are pressed into bilayer tablets and coated.

## Claims

1. A pharmaceutical combination comprising olmesartan medoxomil, amlodipine or a pharmaceutically acceptable salt thereof and erythritol together with one or more pharmaceutically acceptable excipients.

2. The pharmaceutical combination according to claim 1, wherein the pharmaceutical acceptable salt of amlodipine is besylate.

3. The pharmaceutical combination according to claim 1 and 2, wherein the ratio of olmesartan medoxomil to amlodipine besylate is in the range of 0.1 to 10 (w/w) and preferably 0.1 to 6 (w/w).

4. The pharmaceutical combination according to claim 1, wherein the amount of erythritol is between 35 - 90.0%, preferably 35 - 70% and more preferably it is 40.0 - 55.0% by weight of total formulation

5. The pharmaceutical combinationaccording to claim 1, wherein one or more pharmaceutically acceptable excipient is crospovidone.

6. The pharmaceutical combination according to claim 1 and 5, wherein the amount of crospovidone is between 2.0 - 25.0%, preferably 2.0 - 15.0% and more preferably it is 9.0 - 12.0% by weight of total formulation

7. The pharmaceutical combination according to claim 1, further comprising a diuretic.

8. The pharmaceutical combination according to claim 7, wherein said diuretic is hydrochlorothiazide.

9. The pharmaceutical combination according to claim 8, the amount of hydrochlorothiazide is between 1.0 - 15.0%, preferably 1.0 - 10.0% and more preferably it is 2.5 - 6.5% by weight of total formulation.

10. The pharmaceutical combination according to any preceding claim, in the form of a monolayer, bilayer, trilayer, or a multilayer tablet.

11. The pharmaceutical combination according to claim 10, in the form of monolayer tablet.

12. The pharmaceutical combination according to any preceding claim comprising;
a. 0.1 - 15.0 % by weight of olmesartan medoxomil,
b. 0.1 - 15.0 % by weight of amlodipine besylate,
c. 35.0 - 90.0 % by weight of erythritol,
d. 2.0 - 25.0 % by weight of crospovidone,
e. 4.0 - 50.0 % by weight of microcrystalline cellulose,
f. 0.1 - 10.0 % by weight of polyvinylpyrrolidone,
g. 5.0 - 15.0 % by weight of sodium starch glycolate,
h. 0.1 - 5.0 % by weight of colloidal silicon dioxide,
i. 0.1 - 5.0 % by weight of sodium stearyl fumarate,
j. 1.0 - 7.0 % by weight of coating.

13. The pharmaceutical combination according to any preceding claim comprising;
a. 0.1 - 15.0 % by weight of olmesartan medoxomil,
b. 0.1 - 15.0 % by weight of amlodipine besylate,
c. 0.1 - 15.0% by weight of hydrochlorothiazide,
d. 35.0 - 90.0 % by weight of erythritol,
e. 2.0 - 25.0 % by weight of crospovidone,
f. 4.0 - 50.0 % by weight of microcrystalline cellulose,
g. 0.1 - 10.0 % by weight of polyvinylpyrrolidone,
h. 5.0 - 15.0 % by weight of sodium starch glycolate,
i. 0.1 - 5.0 % by weight of colloidal silicon dioxide,
j. 0.1 - 5.0 % by weight of sodium stearyl fumarate,
k. 1.0 - 7.0 % by weight of coating.

14. The pharmaceutical combination according to claim 10, in the form of bilayer tablet.

15. The pharmaceutical combination according to claim 14 comprising; In first layer;
a. 0.1 - 15.0 % by weight of olmesartan medoxomil,
b. 2.0 - 15.0 % by weight of crospovidone,
c. 0.1 - 10.0 % by weight of polyvinylpyrrolidone,
d. 35.0 - 90.0 % by weight of erythritol,
e. 4.0 - 50.0 % by weight of microcrystalline cellulose,
f. 0.1 - 5.0 % by weight of sodium stearyl fumarate In second layer;
g. 0.1 - 15.0 % by weight of amlodipine besylate,
h. 2.0 - 15.0 % by weight of crospovidone,
i. 4.0 - 50.0 % by weight of microcrystalline cellulose,
j. 5.0 - 15.0 % by weight of sodium starch glycolate ,
k. 0.1 - 5.0 % by weight of colloidal silicon dioxide
l. 0.1 - 5.0 % by weight of sodium stearyl fumarate and,
m. 1.0 - 7.0 % by weight of coating for the total tablet.

16. The pharmaceutical combination according to claim 14 comprising; In first layer;
a. 0.1 - 15.0 % by weight of olmesartan medoxomil,
b. 2.0 - 15.0 % by weight of crospovidone,
c. 0.1 - 10.0 % by weight of polyvinylpyrrolidone,
d. 35.0 - 90.0 % by weight of erythritol,
e. 4.0 - 50.0 % by weight of microcrystalline cellulose,
f. 0.1 - 5.0 % by weight of sodium stearyl fumarate In second layer;
g. 0.1 - 15.0 % by weight of amlodipine besylate
h. 0.1 - 15.0% by weight of hydrochlorothiazide,
i. 2.0 - 15.0 % by weight of crospovidone,
j. 4.0 - 50.0 % by weight of microcrystalline cellulose,
k. 5.0 - 15.0 % by weight of sodium starch glycolate ,
l. 0.1 - 5.0 % by weight of colloidal silicon dioxide
m. 0.1 - 5.0 % by weight of sodium stearyl fumarate and,
n. 1.0 - 7.0 % by weight of coating for the total tablet.
